# EUROPÄISCHE PATENTSCHRIFT

(11) **EP 3 702 016 B1**
(45) Veröffentlichungstag und Bekanntmachung des Hinweises auf die Patenterteilung: **05.01.2022**
(21) Anmeldenummer: 19159425.8
(22) Anmeldetag: 26.02.2019
(51) Int. Cl.: B01D 53/22, B01J 8/00, C07C 5/32, C07C 7/144, C07C 11/06

(54) **VERFAHREN UND ANLAGE ZUR GEWINNUNG EINES PROPYLENPRODUKTS**
METHOD AND SYSTEM FOR OBTAINING A PROPYLENE PRODUCT
PROCÉDÉ ET INSTALLATION PERMETTANT D'OBTENIR UN PRODUIT DE PROPYLÈNE

(43) Veröffentlichungstag der Anmeldung: 02.09.2020
(73) Patentinhaber: Linde GmbH, 82049 Pullach (DE)
(72) Erfinder: Fritz, Helmut, 81375 München (DE)

(56) Entgegenhaltungen:
- EP-A1- 2 505 573
- WO-A1-2016/140574

## Beschreibung

Die Erfindung betrifft ein Verfahren und eine Anlage zur Herstellung eines Propylenprodukts gemäß den Oberbegriffen der unabhängigen Patentansprüche

### Stand der Technik

Propylen (Propen) wird herkömmlicherweise überwiegend durch Dampfspalten (engl. Steam Cracking) von Kohlenwasserstoffeinsätzen und weitere Umwandlungsverfahren im Zuge von Raffinerieprozessen hergestellt. In diesen Fällen stellt Propylen ein Nebenprodukt dar, das allerdings nur in vergleichsweise geringer Menge anfällt. Aufgrund des zunehmenden Bedarfs an Propylen, insbesondere für Polypropylen, wird daher auch die Propandehydrierung eingesetzt.

Die (katalytische) Propandehydrierung ist ein allgemein bekanntes Verfahren der petrochemischen Industrie und wird beispielsweise im Artikel "Propene" in Ullmann's Encyclopedia of Industrial Chemistry, Onlineausgabe 16. September 2013, DOI: 10.1002/14356007.a22_211.pub3, insbesondere Abschnitt 3.3.1, "Propane Dehydrogenation", und in WO2016/140574 beschrieben.

Bei der Propandehydrierung handelt es sich um eine endotherme Gleichgewichtsreaktion, die im Allgemeinen an Edel- oder Schwermetallkatalysatoren, umfassend beispielsweise Platin oder Chrom, durchgeführt wird. Die Dehydrierungsreaktion ist ausgesprochen selektiv. Für kommerziell verfügbare Prozesse werden Gesamtausbeuten von ca. 90% genannt. Ungeachtet dieser hohen Selektivität entstehen neben dem abgespaltenen Wasserstoff jedoch typischerweise auch geringere Mengen an Kohlenwasserstoffen mit einem, zwei sowie vier und mehr als vier Kohlenstoffatomen als Nebenprodukte. Diese Nebenprodukte sowie bei der Propandehydrierung nicht umgesetztes Propan müssen zur Gewinnung eines Propylenprodukts abgetrennt werden.

Dampfspaltverfahren und Raffinerieprozesse, in denen Propylen gebildet wird, sind ebenfalls umfangreich in der Literatur beschrieben, beispielsweise im Artikel "Ethylene" in Ullmann's Encyclopedia of Industrial Chemistry, Onlineveröffentlichung 15. April 2009, DOI: 10.1002/14356007.a10_045.pub3, und im Artikel "Oil Refining" in Ullmann's Encyclopedia of Industrial Chemistry, Onlineveröffentlichung 15. Januar 2007, DOI: 10.1002/14356007.a18_051.pub2.

Auch in Dampfspaltverfahren und Raffinerieprozessen werden Propylen enthaltende Komponentengemische gebildet, die zur Gewinnung eines Propylenprodukts entsprechend bearbeitet werden müssen. Die vorliegende Erfindung eignet sich grundsätzlich für alle Verfahren und Anlagen, in denen Komponentengemische gebildet werden, die Propylen sowie insbesondere tiefer als Propylen siedende Komponenten enthalten.

In kommerziellen Anlagen zur Propandehydrierung werden bei der Bearbeitung eines entsprechenden Komponentengemischs nach einer entsprechenden Vorbereitung, beispielsweise einer Verdichtung und Kohlendioxidentfernung, typischerweise zunächst Kohlenwasserstoffe mit zwei Kohlenstoffatomen und leichter siedende Verbindungen von Kohlenwasserstoffen mit drei und mehr Kohlenstoffatomen kryogen abgetrennt (sogenannte Deethanisierung). Anschließend erfolgt, falls vorhanden, ggf. eine Abtrennung von Kohlenwasserstoffen mit drei Kohlenstoffatomen und die Auftrennung der verbleibenden, überwiegend oder ausschließlich Propan und Propylen enthaltenden Fraktion (sogenannte P-P-Trennung). Vergleichbare Verfahren kommen auch zur Bearbeitung von Komponentengemischen zum Einsatz, die in Dampfspaltverfahren und Raffinerieprozessen gebildet werden.

Die aus dem Stand der Technik bekannten kryogenen Verfahren zur Bearbeitung entsprechender Komponentengemische sind durch einen ausgesprochen hohen Investitionsaufwand gekennzeichnet, der insbesondere aus den Maschinen zur Kälteerzeugung und die Apparate für die Kälteübertragung resultiert. Aufgrund des festzustellenden (positiven) Skaleneffekts (engl. Economy of Scale) handelt es sich bei den kryogenen Verfahren für Anlagen großer Kapazität um die Trennverfahren der Wahl. Für Anlagen geringerer Kapazität erhöhen sich die spezifischen Investitionskosten für kryogene Verfahren jedoch unverhältnismäßig.

Es besteht daher der Bedarf nach Alternativen zur trenntechnischen Bearbeitung von Propylen und tiefer als Propylen siedende Komponenten enthaltenden Komponentengemischen, insbesondere wenn diese Komponentengemische in einer Propandehydrierung und in vergleichsweise geringen Mengen anfallen.

### Offenbarung der Erfindung

Vor diesem Hintergrund schlägt die vorliegende Erfindung ein Verfahren und eine Anlage zur Herstellung von Propylen mit den jeweiligen Merkmalen der unabhängigen Patentansprüche vor. Bevorzugte Ausgestaltungen sind Gegenstand der abhängigen Patentansprüche sowie der nachfolgenden Beschreibung.

Fluide beliebiger Art werden hier generell als "reich" an einer oder mehreren enthaltenen Komponenten bezeichnet, wenn der Gehalt an der einen oder den mehreren Komponenten wenigstens 50%, 60%, 70%, 80%, 90%, 95%, 99% oder 99,5% auf molarer, Gewichts- oder Volumenbasis beträgt. Sie werden hingegen als "arm" an der einen oder den mehreren Komponenten bezeichnet, wenn der Gehalt an der einen oder den mehreren Komponenten höchstens 30%, 20%, 10%, 5%, 1% oder 0,5% auf molarer, Gewichts- oder Volumenbasis beträgt. Sie können im hier verwendeten Sprachgebrauch "angereichert" oder "abgereichert" an einer oder mehreren Komponenten sein, wobei sich diese Begriffe auf einen entsprechenden Gehalt in einem Ausgangsfluid beziehen, aus dem das jeweilige Fluid gebildet wurde. Das Fluid wird als angereichert bezeichnet, wenn es zumindest den 2-fachen, 5-fachen, 10-fachen, 100-fachen oder 1.000-fachen Gehalt der einen oder der mehreren Komponenten, bezogen auf das Ausgangsfluid, aufweist. Das Fluid wird hingegen als abgereichert bezeichnet, wenn es höchstens den 0,5-fachen, 0,1-fachen, 0,01-fachen oder 0,001-fachen Gehalt der einen oder der mehreren Komponenten, bezogen auf das Ausgangsfluid, aufweist. Ein "überwiegend" eine oder mehrere Komponenten enthaltendes Fluid ist reich an dieser oder diesen im Sinne der obigen Definition.

Ein Fluid (die Bezeichnung Fluid wird stellvertretend auch für entsprechende Ströme, Fraktionen usw. verwendet) ist im hier verwendeten Sprachgebrauch von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Stoffstrom kann auch beispielsweise einfach dadurch gebildet werden, dass er aus einem Speicherbehälter abgezogen bzw. von einem anderen Stoffstrom abgetrennt wird.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Die vorliegende Erfindung beruht insbesondere auf der Erkenntnis, dass für bekannte Membrantrennverfahren (praktisch) kein Skaleneffekt festzustellen ist, weshalb sie im Allgemeinen für hohe Anlagenkapazitäten keine wirtschaftlichen Lösungen darstellen, dagegen für kleine Anlagen oft kostengünstige Lösungen sind. Membrantrennverfahren unterscheiden sich grundsätzlich von kryogenen Destillationsverfahren typischerweise dahingehend, dass sie nur für Grob-(Bulk-)Abtrennungen geeignet sind, aber keinen hochreinen Produkte liefern können.

Die vorliegende Erfindung schlägt nun vor diesem Hintergrund ein einfaches und kostengünstiges Verfahren zur Abtrennung einer propylenhaltigen Fraktion bzw. eines Propylenprodukts aus einem unter Einsatz einer Propandehydrierung gebildeten Komponentengemisch vor, wobei das Komponentengemisch insbesondere in vergleichsweise geringer Produktionsmenge gebildet wird bzw. die Propandehydrierung eine vergleichsweise geringe Produktionskapazität besitzt. Unter einer "geringen Produktionsmenge" bzw. einer "geringen Produktionskapazität" wird dabei hier eine Produktionsmenge von 20 bis 120 kta (Kilotonnen pro Jahr), insbesondere 35 bis 80 kta, beispielsweise ca. 50 kta, verstanden.

Die im Rahmen der vorliegenden Erfindung vorgeschlagene Trennung erweist sich insbesondere hinsichtlich der Investitionskosten für entsprechend geringe Produktionskapazitäten als deutlich vorteilhafter als herkömmliche kryogene Trennsequenzen, da auf aufwendige Apparate und Maschinen verzichtet werden kann. Zugleich kann, wie auch nachfolgend noch im Detail erläutert, im Rahmen der vorliegenden Erfindung in der Trennung ein Reaktionseinsatz bereitgestellt werden, der Propan und Wasserstoff in für die Propandehydrierung geeigneten Mengen aufweist. Auf externe Zudosierungen kann daher verzichtet werden.

Die vorliegende Erfindung eignet sich insbesondere für die Verwendung in kleineren Propandehydrierungsanlagen, die zur Produktionssteigerung in bestehenden Anlagen, beispielsweise Anlagen zum Dampfspalten, nachgerüstet werden können. Aufgrund der erfindungsgemäß vorgeschlagenen Wasserstoffabtrennung können die verbleibenden Gasgemische in derartigen vorhandenen Anlagen bzw. den dort bereitgestellten Trenneinrichtungen bearbeitet werden und überlasten diese nicht. Es sind daher keine weiteren Umrüstungsmaßnahmen erforderlich.

Die vorliegende Erfindung schlägt insgesamt ein Verfahren zur Herstellung eines Propylenprodukts vor, bei dem unter Einsatz einer Propandehydrierung, der ein Propan und Wasserstoff enthaltender Reaktionseinsatz unterworfen wird, ein Propan, Propylen und Wasserstoff enthaltendes Komponentengemisch bereitgestellt wird. Wie bereits zuvor angesprochen, kann das erfindungsgemäße Verfahren dabei insbesondere in ein Gesamtverfahren bzw. eine Anlage integriert werden, in der weitere stoffumwandelnde Verfahrensschritte und Trennschritte durchgeführt werden, beispielsweise ein oder mehrere Dampfspaltschritte und diesem zugeordnete Trennschritte. Das erfindungsgemäß hergestellte Propylenprodukt kann dabei insbesondere auch unter Verwendung entsprechender weiterer Trennschritte gewonnen werden.

Die vorliegende Erfindung sieht vor, dass das Komponentengemisch oder ein Teil hiervon als ein erster Trenneinsatz einer ersten Membrantrennung unterworfen wird, mittels derer ein gegenüber dem ersten Trenneinsatz an Wasserstoff angereichertes erstes Permeat sowie ein gegenüber dem ersten Trenneinsatz an Wasserstoff abgereichertes, Wasserstoff, Propan und Propylen enthaltendes erstes Retentat gebildet werden. Das erste Permeat enthält dabei vorteilhafterweise kein oder nur geringe Mengen an Propan und Propylen, die in dem der ersten Membrantrennung zugeführten ersten Trenneinsatz enthalten sind. Wie erwähnt, sind Membrantrennverfahren typischerweise nicht dafür eingerichtet, eine vollständige Abtrennung von Komponenten zu erzielen, so dass das erste Retentat noch beträchtliche Mengen des in dem ersten Trenneinsatz enthaltenen Wasserstoffs enthält. Für dessen weitere Abtrennung wird die nachfolgend noch erläuterte zweite Membrantrennung verwendet.

Die erste (und die nachfolgend noch erläuterte zweite) Membrantrennung kann bzw. können im Rahmen der vorliegenden Erfindung unter Verwendung von aus dem Stand der Technik grundsätzlich bekannten Membrantrenneinrichtungen durchgeführt werden. Zu weiteren Details sei auf Fachliteratur verwiesen.

Unter einem "Permeat" wird hier ein Gas oder Gasgemisch verstanden, das überwiegend oder ausschließlich Komponenten aufweist, die von einer in einer Membrantrennstufe eingesetzten Membran nicht oder überwiegend nicht zurückgehalten werden, die also die Membran (im Wesentlichen oder zumindest bevorzugt) ungehindert passieren. Entsprechend handelt es sich bei einem "Retentat" um ein Gas oder Gasgemisch, das überwiegend oder ausschließlich Komponenten aufweist, die von der in der Membrantrennstufe eingesetzten Membran vollständig oder zumindest überwiegend zurückgehalten werden.

Im Rahmen der vorliegenden Erfindung wird das erste Retentat oder ein Teil hiervon als zweiter Trenneinsatz einer zweiten Membrantrennung unterworfen, in der ein zumindest den überwiegenden Teil des Wasserstoffs des zweiten Trenneinsatzes enthaltendes zweites Permeat sowie ein zumindest den überwiegenden Teil des Propans und des Propylens des zweiten Trenneinsatzes enthaltendes zweites Retentat gebildet werden. In der zweiten Membrantrennung wird, mit anderen Worten, der Wasserstoff aus dem zweiten Trenneinsatz überwiegend oder vollständig entfernt, so dass ein vorteilhafterweise im Wesentlichen schwerere Komponenten, insbesondere Propan und Propylen, enthaltendes Retentat gebildet wird. Dieses zweite Retentat kann dann zur Gewinnung des Propylenprodukts weiter aufbereitet werden.

Im Rahmen der vorliegenden Erfindung können grundsätzlich zwei unterschiedliche, je nach den vorliegenden Gegebenheiten vorteilhafte Alternativen zum Einsatz kommen. So kann die erste Membrantrennung unter Einsatz eines Propan enthaltenden Spülgases durchgeführt werden und das erste Permeat damit als mit Propan des Spülgases beaufschlagtes Permeat erhalten werden. Ein entsprechendes Spülgas wird in der Membrantrennung auf der Permeatseite entlang der verwendeten Membran geführt und ermöglicht es, eine größere Menge bzw. einen größeren Anteil über die Membran zu gewinnen, da durch die Membran tretende leichtere Komponenten kontinuierlich abgeführt werden können und damit ein Konzentrations- bzw. Partialdruckgefälle über die Membran hinweg aufrecht erhalten werden kann. Als das Propan enthaltende Spülgas wird dabei insbesondere Propan oder ein an Propan reiches, jedenfalls aber vorteilhafterweise an Wasserstoff armes oder freies, Gas oder Gasgemisch verwendet, um die genannten vorteilhaften Effekte in der Membrantrennung zu erzielen. In jedem Fall stammt das Propan in dem mit Propan des Spülgases beaufschlagten Permeat aus dem Spülgas, und nicht oder nur zu geringen Anteilen aus dem ersten Trenneinsatz.

Alternativ oder zusätzlich ist es im Rahmen der vorliegenden Erfindung aber auch möglich, die zweite Membrantrennung unter Einsatz des Propan enthaltenden Spülgases durchzuführen wird und damit das zweite Permeat als mit Propan des Spülgases beaufschlagtes Permeat zu erhalten wird. Hierzu gilt im Wesentlichen das bereits zu dem Einsatz des Propan enthaltenden Spülgases in der ersten Membrantrennung Gesagte.

Im Rahmen der vorliegenden Erfindung ist schließlich vorgesehen, das mit Propan des Spülgases beaufschlagte erste Permeat und/oder das mit Propan des Spülgases beaufschlagte zweite Permeat oder ein oder mehrere Teile hiervon bei der Bildung des Reaktionseinsatzes zu verwenden. Wird eines der Permeate, d.h. das erste oder das zweite Permeat, hingegen nicht als mit Propan des Spülgases beaufschlagtes Permeat gewonnen, wird also in der entsprechenden ersten oder zweiten Membrantrennung kein entsprechendes Spülgas eingesetzt, wird dieses typischerweise nicht bei der Bildung des Reaktionseinsatzes verwendet sondern, wie auch nachfolgend noch erläutert, insbesondere in einem oder mehreren Reaktoren unterfeuert bzw. einem Brenngasnetzwerk in einer entsprechenden Anlage zugeführt.

Der besondere Vorteil, ein mit Propan eines Propan enthaltenden Spülgases beaufschlagtes Permeat bei der Bildung des Reaktionseinsatzes zu verwenden, besteht darin, dass das entsprechende mit Propan beaufschlagte Permeat bereits die in einem entsprechenden Reaktionseinsatz benötigten Komponenten, Propan und Wasserstoff, enthält, und damit keine separate Zudosierung erfolgen muss. Im Rahmen der vorliegenden Erfindung ergibt durch die Verwendung des Spülgases der doppelte Vorteil, dass die entsprechende Membrantrennung vorteilhaft beeinflussen und zugleich ein für einen Einsatz in einem Trenneinsatz direkt verwendbares Gasgemisch bereitstellen lässt. Insbesondere kann, weil der in einem entsprechenden Reaktionseinsatz in der erfindungsgemäßen Weise bereitgestellte Wasserstoff nicht aus externen Quellen sondern aus einer Membrantrennung stammt, auf zusätzliche, ggf. ansonsten erforderliche Reinigungsschritte verzichtet werden.

Im Rahmen der vorliegenden Erfindung enthält der der Propandehydrierung unterworfene Reaktionseinsatz insbesondere 45 bis 95 Volumenprozent Propan und 1 bis 50 Volumenprozent, insbesondere 5 bis 40 Volumenprozent, beispielsweise 9 bis 30 Volumenprozent, Wasserstoff. Ein entsprechender Wasserstoffanteil kann vorteilhafterweise teilweise oder vollständig durch den erfindungsgemäß in dem oder den Membrantrennnschritten in das entsprechend verwendete Permeat übergehenden Wasserstoff gedeckt werden.

Das in der Propandehydrierung gebildete Komponentengemisch und der erste Trenneinsatz enthalten vorteilhafterweise 40 bis 60 Volumenprozent Propan, 20 bis 30 Volumenprozent Propylen und 25 bis 35 Volumenprozent Wasserstoff. In der Propandehydrierung wird damit zusätzlicher (überschüssiger) Wasserstoff gebildet, der in dem Anteil, in dem er nicht in die Propandehydrierung zurückgeführt wird, in vorteilhafter Weise für andere Zwecke genutzt werden kann.

In der ersten Membrantrennung erfolgt, wie bereits erwähnt, eine teilweise Abreicherung des ersten Trenneinsatzes an Wasserstoff, so dass das das erste Retentat und der zweite Trenneinsatz im Rahmen der vorliegenden Erfindung insbesondere 10 bis 20 Volumenprozent Wasserstoff aufweisen.

Durch den Einsatz der zweiten Membrantrennung erfolgt eine weitere Wasserstoffabreicherung, so dass das zweite Retentat nur noch geringe Anteile von Wasserstoff von 5 bis 15 Volumenprozent Wasserstoff aufweist.

Vorteilhafterweise wird in dem erfindungsgemäßen Verfahren das Komponentengemisch oder dessen der ersten Membrantrennung unterworfener Teil einer Abkühlung und Verdichtung unterworfen, bevor es oder er der Membrantrennung unterworfen wird. Die Abkühlung erfolgt dabei insbesondere durch Wärmetausch mit dem Reaktionseinsatz oder einem Teil hiervon, bevor dieser der Propandehydrierung unterworfen wird. In diese Ausgestaltung erlaubt das erfindungsgemäß vorgeschlagene Verfahren eine besonders vorteilhafte Wärmerückgewinnung.

Die Verdichtung des Komponentengemischs oder dessen der ersten Membrantrennung unterworfenen Teils erfolgt im Rahmen der vorliegenden Erfindung vorteilhafterweise auf ein Druckniveau von 2 bis 38 bar, 6 bis 20 bar, 8 bis 18 bar, 10 bis 14 bar, 10 bis 16 bar oder 12 bis 14 bar. Dieses Druckniveau richtet sich insbesondere nach dem erforderlichen Abgabedruck des zweiten Retentats bzw. auf einem erforderlichen Eintrittsdruck des oder der Verfahrensschritte, dem oder denen das zweite Retentat unterworfen werden soll. Das Druckniveau richtet sich ferner nach dem Volumenanteil des Wasserstoffs in dem Komponententgemisch aus der Propandehydrierung, weil sich der vorteilhafte Abtrenndruck in der ersten und der zweiten Membrantrennung wiederum von einem entsprechenden Volumenanteil abhängig. Ferner wird das Druckniveau dann, wenn in der ersten und der zweiten Membrantrennung Wasserstoff oder eine wasserstoffreiche Fraktion als Permeat gewonnen wird, der Wärmegewinnung verfeuert werden soll, auch nach dem hierzu erforderlichen Druck, insbesondere dann, wenn eine Einspeisung in ein Heizgasnetzwerk erfolgen soll. Ein weiterer Einflussfaktor ist die Außentemperatur, weil Kohlenwasserstoffe mit drei und ggf. mehr Kohlenstoffatomen in der ersten und der zweiten Membrantrennung nicht kondensieren sollen. Je nach der Ausgestaltung der in dem ersten und dem zweiten Membrantrennschritt eingesetzten Membranen kann in Abhängigkeit vom Partialdruck entsprechender Komponenten auch eine Begleitheizung vorteilhaft sein. Schließlich ist ein weiterer druckbeeinflussender Faktor der verwendete Verdichter. Vorteilhafterweise sollte dieser nicht mehr als zwei Zwischenkühlstufen aufweisen.

Als Verdichter wird vorteilhafterweise ein mit einem Elektromotor angetriebener Verdichter, insbesondere ein dreistufiger Turboverdichter, eingesetzt, da durch den elektrischen Antrieb aufwendige Turbinen und eine ebenso aufwendige Dampfsystemanbindung eingespart werden können.

Die Einspeisung des zweiten Retentats in dem oder die weiteren Verfahrensschritte, insbesondere zur Dampfspaltung oder einem oder mehreren dieser nachgeschalteten Trennschritten, erfolgt vorteilhafterweise auf einem Druckniveau von 1 bis 38 bar, so dass bei geeigneter Wahl der Verdichtung des Rohgases aus der Propandehydrierung keine weiteren Verdichtungsschritte erforderlich sind.

Insbesondere kann im Rahmen der vorliegenden Erfindung nur die erste Membrantrennung unter Einsatz des Propan enthaltenden Spülgases durchgeführt werden und damit nur das erste Permeat als ein mit Propan des Spülgases beaufschlagtes Permeat erhalten werden. Alternativ dazu kann nur die zweite Membrantrennung unter Einsatz des Propan enthaltenden Spülgases durchgeführt werden und nur das zweite Permeat als mit Propan des Spülgases beaufschlagtes Permeat erhalten werden. In beiden Fällen kann das jeweils nicht mit Propan des Spülgases beaufschlagte Permeat oder ein Teil hiervon in einem oder mehreren für die Propandehydrierung verwendeten Reaktoren zur Beheizung verfeuert werden. Hierzu kann beispielsweise eine Einspeisung in ein Heizgasnetzwerk erfolgen, wie bereits erwähnt. Alternativ dazu ist auch eine andere Nutzung eines entsprechenden, insbesondere überwiegend oder ausschließlich Wasserstoff aufweisenden Permeats möglich. In jedem Fall kann auf diese Weise der in der Propandehydrierung gebildete Wasserstoff einer vorteilhaften Verwendung zugeführt werden.

Des jeweils nicht mit Propan des Spülgases beaufschlagte Permeat wird für eine entsprechende Verwendung in der jeweiligen ersten oder zweiten Membrantrennung vorteilhafterweise auf einem Druckniveau von 3 bis 7 bar bereitgestellt und kann auf diese Weise insbesondere in ein Heizgasnetzwerk, das auf einem entsprechenden Druckniveau betrieben wird, eingespeist werden.

Die vorliegende Erfindung stellt auch eine Anlage zur Verfügung, deren Merkmale in dem entsprechenden unabhängigen Patentanspruch angegeben sind. Zu Merkmalen und Vorteilen der erfindungsgemäß vorgeschlagenen Anlage sei auf die zuvor bereits bezüglich des erläuterten Verfahrens und seiner vorteilhaften Ausgestaltung in erläuterten Merkmale und Vorteile ausdrücklich verwiesen. Entsprechendes gilt insbesondere für eine Anlage gemäß einer besonders bevorzugten Ausführungsformen der vorliegenden Erfindung, welche Mittel aufweist, die zur Durchführung eines entsprechenden Verfahrens eingerichtet sind.

Die Erfindung wird nachfolgend unter Bezugnahme auf die beigefügte Zeichnungen näher erläutert, in der eine bevorzugte Ausführungsform der vorliegenden Erfindung veranschaulicht ist.

### Kurze Beschreibung der Zeichnungen

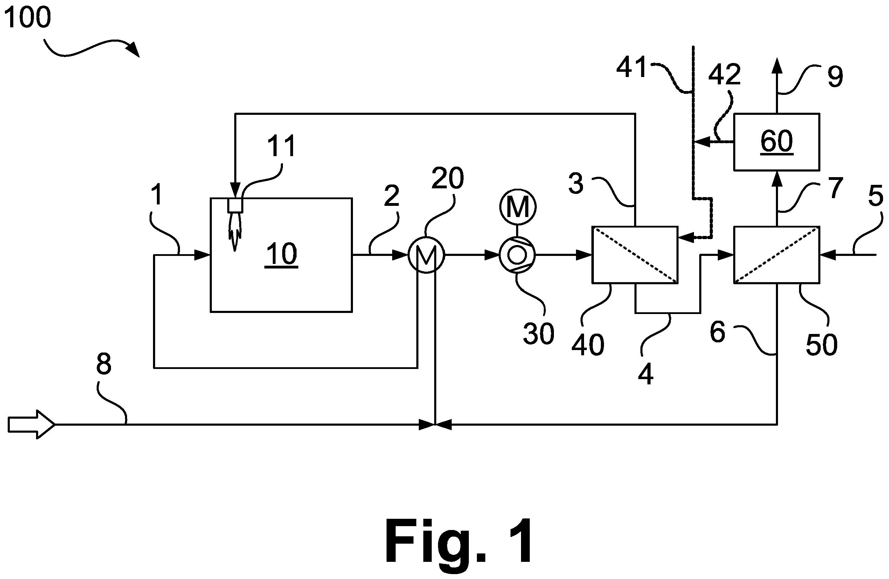

Figur 1 veranschaulicht einen gemäß einer Ausführungsform der Erfindung ausgestaltetes Verfahren in stark vereinfachter, schematischer Darstellung.

### Ausführliche Beschreibung der Zeichnungen

In Figur 1 ist ein erfindungsgemäß ausgestaltetes Verfahren in stark vereinfachter, schematischer Darstellung veranschaulicht und insgesamt mit 100 bezeichnet.

In dem Verfahren 100 werden ein oder mehrere Reaktoren eingesetzt, der oder die für eine Propandehydrierung 10 eingerichtet ist oder sind, und dem oder denen ein Propan enthaltender Reaktionseinsatz 1 zugeführt wird. Der oder die Reaktoren wird oder werden mit einem oder mehreren Brennern 11 beheizt.

Mittels des oder der Reaktoren, und damit unter Einsatz der darin durchgeführten Propandehydrierung 10, wird ein Propan, Propylen und Wasserstoff enthaltendes Komponentengemisch 2 gebildet und damit in dem Verfahren 100 bereitgestellt. Die Produktionsmenge beträgt beispielsweise ca. 50 kta. Das Komponentengemisch 2, oder nur ein Teil des Komponentengemischs 2, wird einer Abkühlung 20 und dabei insbesondere ein Wärmetausch mit dem Einsatzgemisch 1 unterworfen.

Nach der Abkühlung 20 bzw. Abkühlung wird das Komponentengemisch 2 oder dessen der Abkühlung unterworfener Teil, oder wiederum nur ein Teil hiervon, einer Verdichtung 30 unterworfen, wobei insbesondere ein mittels eines Elektromotors M angetriebener dreistufiger Turboverdichter in einem die drei Verdichterstufen einschließenden Gehäuse zum Einsatz kommen kann. Die Verdichtung erfolgt insbesondere auf ein Druckniveau von 12 bis 15 bar.

Nach der Verdichtung 30 wird das Komponentengemisch oder dessen der Verdichtung 30 unterworfener Teil, oder wiederum ein nur Teil hiervon, als erster Trenneinsatz einer ersten Membrantrennung 40 unterworfen. In dieser werden im dargestellten Beispiel ein gegenüber dem ersten Trenneinsatz an Wasserstoff angereichertes und an Propan und Propylen armes oder freies erstes Permeat 3 und ein gegenüber dem ersten Trenneinsatz an Wasserstoff abgereichertes, jedoch noch Wasserstoff und ansonsten Propan und Propylen enthaltendes erstes Retentat 4 gebildet. Im dargestellten Beispiel wird die erste Membrantrennung ohne Verwendung eines Propan enthaltenden Spülgases durchgeführt und damit das erste Permeat 3 nicht als ein mit Propan eines Spülgases beaufschlagtes Permeat gewonnen. Wie erläutert, werden aber auch Verfahrensvarianten in Erwägung gezogen, in denen genau dies erfolgt. Wie in Form eines Stoffstroms 41 veranschaulicht, kann optional eine Spülung mit anderen Medien, beispielsweise Erdgas, Stickstoff und/oder Kohlenwasserstoffen mit Komponenten aus unten erläuterten weiteren Verfahrensschritten 60, insbesondere Kohlenwasserstoffen mit zwei und weniger Kohlenstoffatomen und ggf. leichteren Verbindungen, wie in Form eines Stoffstroms 42 veranschaulicht, erfolgen.

Die Membrantrennung 40 wird dabei im dargestellten Beispiel insbesondere derart vorgenommen, dass ein Druckniveau, auf dem das erste Permeat 3 gewonnen wird, gerade hoch genug ist, dass das erste Permeat 3, oder nur ein Teil hiervon, ohne weitere Verdichtung dem oder den Brennern 11 zugeführt und in diesem oder diesen als Heizgas eingesetzt werden kann. Reicht eine Menge des ersten Permeats 3 für eine Beheizung mittels des oder der Brenner 11 nicht aus, kann insbesondere vorgesehen sein, in der ersten Membrantrennung 30 eine Spülung mit einem brennbaren Spülgas, insbesondere Methan bzw. einem Methan enthaltenden Gasgemisch, insbesondere mit Erdgas, vorzunehmen. Das erste Permeat 3 kann auch in ein Heizgasnetz einer entsprechenden Anlage oder eines entsprechenden Anlagenverbunds eingespeist werden.

Das erste Retentat 4, oder nur ein Teil hiervon, wird als zweiter Trenneinsatz einer zweiten Membrantrennung 50 unterworfen, in der ein gegenüber dem zweiten Trenneinsatz unter Einsatz eines Propan enthaltenden Spülgases 5 ein zumindest den überwiegenden Anteil des in dem zweiten Trenneinsatz enthaltenen Wasserstoffs enthaltendes zweites Permeat 6 und ein zumindest den überwiegenden Teil des Propans und des Propylens aus dem zweiten Trenneinsatz enthaltendes zweites Retentat 7 gebildet werden. Das Spülgas 5 wird insbesondere deshalb verwendet, damit ungeachtet der in der ersten Membrantrennung 40 bereits erfolgten Reduzierung des Wasserstoffpartialdrucks eine ausreichende, d.h. vollständige oder im Wesentlichen vollständige, Wasserstoffabtrennung erfolgen kann.

Das zweite Permeat 6 umfasst aufgrund der erläuterten Bildung in der zweiten Membrantrennung 50 Propan des Spülgases 5 und Wasserstoff aus dem zweiten Trenneinsatz. Im dargestellten Beispiel wird also das zweite Permeat 6 als ein mit Propan des Spülgases 5 beaufschlagtes Permeat gewonnen. Das zweite Permeat 6, oder nur ein Teil hiervon, kann daher in besonders vorteilhafter Weise als Teil des Reaktionseinsatzes 1 in den oder die Reaktoren 10 zurückgeführt werden, da in der dort durchgeführten Propandehydrierung typischerweise Reaktionseinsätze zum Einsatz kommen, die einen bestimmten Anteil an Wasserstoff enthalten. Zu anderen Ausgestaltungen sei auf die obigen Erläuterungen verwiesen.

Daher kann im Rahmen der vorliegenden Erfindung die zweite Membrantrennung sowohl zur Abtrennung von Wasserstoff zur Gewinnung eines Propylenprodukts als auch zur Zudosierung von Wasserstoff zu einem Reaktionseinsatz 1 verwendet werden. Hierbei wird das zweite Permeat 6, oder dem als Teil des Reaktionseinsatzes 1 in den oder die Reaktoren 10 zurückgeführten Teil hiervon insbesondere ein überwiegend oder ausschließlich Propan enthaltender Frischeinsatz 8 zugegeben.

Das zweite Retentat 7 wird im dargestellten Beispiel einem oder mehreren weiteren Verfahrensschritten 60, insbesondere einer Trennung von Propan und Propylen und ggf. einer Abtrennung schwererer Komponenten zugeführt. In dem oder den weiteren Verfahrensschritten 60 kann, neben einer oder mehreren anderen, hier nicht gesondert veranschaulichten weiteren Fraktionen, insbesondere ein überwiegend oder ausschließlich Propylen enthaltendes Propylenprodukt 9 gebildet werden. Ferner kann in dem oder den weiteren Verfahrensschritten 60 insbesondere auch eine überwiegend oder ausschließlich Propan enthaltende Fraktion gewonnen werden, welche als das Spülgas 5 verwendet werden kann. Der oder die weiteren Verfahrensschritte 60 kann bzw. können auch insbesondere Teil eines Dampfspaltprozesses mit entsprechend zugeordneten Trennschritten sein.

## Patentansprüche

1. Verfahren (100) zur Herstellung eines Propylenprodukts (9), bei dem unter Einsatz einer Propandehydrierung (10), der ein Propan und Wasserstoff enthaltender Reaktionseinsatz (1) unterworfen wird, ein Propan, Propylen und Wasserstoff enthaltendes Komponentengemisch (2) bereitgestellt wird,
- wobei das Komponentengemisch (2) oder ein Teil hiervon als ein erster Trenneinsatz einer ersten Membrantrennung (40) unterworfen wird, mittels derer ein gegenüber dem ersten Trenneinsatz an Wasserstoff angereichertes erstes Permeat (3) sowie ein gegenüber dem ersten Trenneinsatz an Wasserstoff abgereichertes, Wasserstoff, Propan und Propylen enthaltendes erstes Retentat (4) gebildet werden,
- wobei das erste Retentat (4) oder ein Teil hiervon als zweiter Trenneinsatz einer zweiten Membrantrennung (50) unterworfen wird, in der ein zumindest den überwiegenden Teil des Wasserstoffs des zweiten Trenneinsatzes enthaltendes zweites Permeat (6) sowie ein zumindest den überwiegenden Teil des Propans und des Propylens des zweiten Trenneinsatzes enthaltendes zweites Retentat (7) gebildet werden,
- wobei die erste Membrantrennung (40) unter Einsatz eines Propan enthaltenden Spülgases (5) durchgeführt wird und das erste Permeat (3) als mit Propan des Spülgases (5) beaufschlagtes Permeat (3) erhalten wird und/oder die zweite Membrantrennung (50) unter Einsatz des Propan enthaltenden Spülgases (5) durchgeführt wird und das zweite Permeat (6) als mit Propan des Spülgases (5) beaufschlagtes Permeat (6) erhalten wird,
- wobei das mit Propan des Spülgases (5) beaufschlagte erste Permeat (3) und/oder das mit Propan des Spülgases beaufschlagte zweite Permeat (6) oder ein oder mehrere Teile hiervon bei der Bildung des Reaktionseinsatzes (1) verwendet wird.

2. Verfahren nach Anspruch 1, bei dem der Reaktionseinsatz (1) 45 bis 95 Volumenprozent Propan und 1 bis 50 Volumenprozent Wasserstoff aufweist.

3. Verfahren nach Anspruch 1 oder 2, bei dem das Komponentengemisch (2) und der erste Trenneinsatz 40 bis 60 Volumenprozent Propan, 20 bis 30 Volumenprozent Propylen und 25 bis 35 Volumenprozent Wasserstoff aufweisen.

4. Verfahren nach einem der vorstehenden Ansprüche, bei dem das erste Retentat und der zweite Trenneinsatz 10 bis 20 Volumenprozent Wasserstoff aufweisen.

5. Verfahren nach einem der vorstehenden Ansprüche, bei dem das zweite Retentat 5 bis 15 Volumenprozent Wasserstoff aufweist.

6. Verfahren nach einem der vorstehenden Ansprüche, bei dem das Komponentengemisch (2) oder dessen der ersten Membrantrennung (40) unterworfener Teil einer Abkühlung (20) und Verdichtung (30) unterworfen wird, bevor es oder er der Membrantrennung (40) unterworfen wird.

7. Verfahren nach Anspruch 6, bei dem die Verdichtung (30) auf ein Druckniveau von 2 bis 38 bar vorgenommen wird.

8. Verfahren nach Anspruch 6 oder 7, bei dem die Verdichtung (30) unter Verwendung eines oder mehrerer mehrstufiger Turboverdichter, insbesondere mit zwei oder weniger Zwischenkühlstufen, vorgenommen wird.

9. Verfahren nach einem der vorstehenden Ansprüche, bei dem das zweite Retentat oder ein Teil hiervon, insbesondere auf einem Druckniveau von 1 bis 38 bar, einem oder mehreren weiteren Verfahrensschritten (60) zugeführt wird.

10. Verfahren nach Anspruch 9, bei dem der oder die weiteren Verfahrensschritte (60) einen Dampfspaltprozess oder einen oder mehrere dem Dampfspaltprozess zugeordnete Trennschritte umfassen.

11. Verfahren nach einem der vorstehenden Ansprüche, bei dem nur die erste Membrantrennung (40) unter Einsatz des Propan enthaltenden Spülgases (5) durchgeführt wird und nur das erste Permeat (3) als mit Propan des Spülgases (5) beaufschlagtes Permeat (3) erhalten wird oder bei dem nur die zweite Membrantrennung (50) unter Einsatz des Propan enthaltenden Spülgases (5) durchgeführt wird und nur das zweite Permeat (6) als mit Propan des Spülgases (5) beaufschlagtes Permeat (6) erhalten wird, wobei das jeweils nicht mit Propan des Spülgases (5) beaufschlagte Permeat (3, 6) oder ein Teil hiervon in einem oder mehreren für die Propandehydrierung (10) verwendeten Reaktoren zur Beheizung verfeuert wird.

12. Verfahren nach Anspruch 11, bei dem das jeweils nicht mit Propan des Spülgases (5) beaufschlagte Permeat (3, 6) in der Membrantrennung (40, 50) auf einem Druckniveau von 3 bis 7 bar bereitgestellt wird.

13. Anlage zur Herstellung eines Propylenprodukts (9), mit einem oder mehreren Reaktoren, der oder die dafür eingerichtet sind, unter Einsatz einer Propandehydrierung (10), dem ein Propan und Wasserstoff enthaltender Reaktionseinsatz (1) unterworfen wird, ein Propan, Propylen und Wasserstoff enthaltendes Komponentengemisch (2) bereitzustellen,
- Mitteln, die dafür eingerichtet sind, das Komponentengemisch (2) oder einen Teil hiervon als einen ersten Trenneinsatz einer ersten Membrantrennung (40) zu unterwerfen, mittels derer ein gegenüber dem ersten Trenneinsatz an Wasserstoff angereichertes erstes Permeat (3) sowie ein gegenüber dem ersten Trenneinsatz an Wasserstoff abgereichertes, Wasserstoff, Propan und Propylen enthaltendes erstes Retentat (4) gebildet werden,
- Mitteln, die dafür eingerichtet sind, das erste Retentat (4) oder einen Teil hiervon als einen zweiten Trenneinsatz einer zweiten Membrantrennung (50) zu unterwerfen, in der ein zumindest den überwiegenden Teil des Wasserstoffs des zweiten Trenneinsatzes und des Propans des Spülgases enthaltendes zweites Permeat (6) sowie ein zumindest den überwiegenden Teil des Propans und des Propylens des zweiten Trenneinsatzes enthaltendes zweites Retentat gebildet werden,
- Mitteln, die dafür eingerichtet sind, die erste Membrantrennung (40) unter Einsatz eines Propan enthaltenden Spülgases (5) durchzuführen und das erste Permeat (3) als mit Propan des Spülgases (5) beaufschlagtes Permeat (3) zu erhalten und/oder die zweite Membrantrennung (50) unter Einsatz des Propan enthaltenden Spülgases (5) durchzuführen und das zweite Permeat (6) als mit Propan des Spülgases (5) beaufschlagtes Permeat (6) zu erhalten,
- Mitteln, die dafür eingerichtet sind, das mit Propan des Spülgases (5) beaufschlagte erste Permeat (3) und/oder das mit Propan des Spülgases beaufschlagte zweite Permeat (3) oder ein oder mehrere Teile hiervon bei der Bildung des Reaktionseinsatzes (1) zu verwendet.

14. Anlage nach Anspruch 13, die zur Durchführung eines Verfahrens nach einem der vorstehenden Ansprüche eingerichtet ist.

## Claims

1. Method (100) for obtaining a propylene product (9), wherein a component mixture (2) containing propane, propylene and hydrogen is provided using propane dehydrogenation (10) to which a reaction feed (1) containing propane and hydrogen is subjected,
- wherein the component mixture (2) or a portion thereof as a first separation feed is subjected to a first membrane separation process (40) by means of which a first permeate (3), which is enriched in hydrogen with respect to the first separation feed, and a first retentate (4), which is depleted in hydrogen with respect to the first separation feed and which contains hydrogen, propane and propylene, are formed,
- wherein the first retentate (4) or a portion thereof as a second separation feed is subjected to a second membrane separation process (50) in which a second permeate (6) containing at least the predominant portion of the hydrogen of the second separation feed and a second retentate (7) containing at least the predominant portion of the propane and of the propylene of the second separation feed are formed,
- wherein the first membrane separation process (40) is carried out using a purge gas (5) containing propane, and the first permeate (3) is obtained as a permeate (3) to which propane of the purge gas (5) is supplied, and/or the second membrane separation process (50) is carried out using the purge gas (5) containing propane and the second permeate (6) is obtained as a permeate (6) to which propane of the purge gas (5) is supplied,
- wherein the first permeate (3) supplied with propane of the purge gas (5) and/or the second permeate (6) supplied with propane of the purge gas or one or more portions thereof is used in the formation of the reaction feed (1).

2. Method according to claim 1, wherein the reaction feed (1) comprises 45 to 95 volume percent propane and 1 to 50 volume percent hydrogen.

3. Method according to claim 1 or 2, wherein the component mixture (2) and the first separation feed comprise 40 to 60 volume percent propane, 20 to 30 volume percent propylene and 25 to 35 volume percent hydrogen.

4. Method according to any one of the preceding claims, wherein the first retentate and the second separation feed comprise 10 to 20 volume percent hydrogen.

5. Method according to any one of the preceding claims, wherein the second retentate comprises 5 to 15 volume percent hydrogen.

6. Method according to any one of the preceding claims, wherein the component mixture (2) or its portion subjected to the first membrane separation process (40) is subjected to cooling (20) and compression (30) before being subjected to the membrane separation process (40).

7. Method according to claim 6, wherein the compression (30) is carried out to a pressure level of 2 to 38 bar.

8. Method according to claim 6 or 7, wherein the compression (30) is carried out using one or more multi-stage turbo-compressors, in particular with two or fewer intermediate cooling stages.

9. Method according to any one of the preceding claims, wherein the second retentate or a portion thereof is supplied to one or more further method steps (60), in particular at a pressure level of 1 to 38 bar.

10. Method according to claim 9, wherein the further method step(s) (60) comprise(s) a steam cracking process or one or more separation steps associated with the steam cracking process.

11. Method according to any one of the preceding claims, wherein only the first membrane separation process (40) is carried out using the purge gas (5) containing propane, and only the first permeate (3) is obtained as a permeate (3) to which propane of the purge gas (5) is supplied, or wherein only the second membrane separation process (50) is carried out using the purge gas (5) containing propane, and only the second permeate (6) is obtained as a permeate (6) to which propane of the purge gas (5) is supplied, wherein the permeate (3, 6) not supplied with propane of the purge gas (5) in each case or a portion thereof is burnt for heating in one or more reactors used for the propane dehydrogenation (10).

12. Method according to claim 11, wherein the permeate (3, 6) not supplied with propane of the purge gas (5) in each case is provided in the membrane separation process (40, 50) at a pressure level of 3 to 7 bar.

13. System for obtaining a propylene product (9), having one or more reactors configured to provide a component mixture (2) containing propane, propylene and hydrogen using propane dehydrogenation (10) to which a reaction feed (1) containing propane and hydrogen is subjected,
- means configured to subject the component mixture (2) or a portion thereof as a first separation feed to a first membrane separation process (40) by means of which a first permeate (3) enriched with hydrogen with respect to the first separation feed and a first retentate (4) depleted of hydrogen with respect to the first separation feed and containing hydrogen, propane and propylene are formed,
- means configured to subject the first retentate (4) or a portion thereof as a second separation feed to a second membrane separation process (50) in which a second permeate (6) containing at least the predominant portion of the hydrogen of the second feed and of the propane of the purge gas and a second retentate containing at least the predominant portion of the propane and of the propylene of the second separation feed are formed,
- means configured to carry out the first membrane separation process (40) using a purge gas (5) containing propane, and to obtain the first permeate (3) as a permeate (3) to which propane of the purge gas (5) is supplied, and/or to carry out the second membrane separation process (50) using the purge gas (5) containing propane, and to obtain the second permeate (6) as a permeate (6) to which propane of the purge gas (5) is supplied,
- means configured to use the first permeate (3) supplied with propane of the purge gas (5) and/or the second permeate (3) supplied with propane of the purge gas, or one or more portions thereof, in the formation of the reaction feed (1).

14. System according to claim 13, configured to carry out a method according to any one of the preceding claims.

## Revendications

1. Procédé (100) de production d'un produit à base de propylène (9), selon lequel, à l'aide d'une déshydrogénation de propane (10) à laquelle une charge réactionnelle contenant du propane et de l'hydrogène (1) est soumise, un mélange de composants contenant du propane, du propylène et de l'hydrogène (2) est fourni,
- où le mélange de composants (2) ou une partie de celui-ci est soumis, en tant que première charge séparatrice, à une première séparation membranaire (40) au moyen de laquelle un premier perméat (3) enrichi en hydrogène par rapport à la première charge séparatrice, ainsi qu'un premier rétentat (4) appauvri en hydrogène par rapport à la première charge séparatrice et contenant de l'hydrogène, du propane et du propylène, sont formés,
- où le premier rétentat (4) ou une partie de celui-ci est soumis, en tant que seconde charge séparatrice, à une seconde séparation membranaire (50), dans laquelle un second perméat (6) contenant au moins la majorité de l'hydrogène de la seconde charge séparatrice, ainsi qu'un second rétentat (7) contenant au moins la majorité du propane et du propylène de la seconde charge séparatrice sont formés,
- où la première séparation membranaire (40) est mise en œuvre à l'aide d'un gaz de balayage (5) contenant du propane et le premier perméat (3) est obtenu sous la forme de perméat (3) chargé en propane du gaz de balayage (5) et/ou la seconde séparation membranaire (50) est mise en œuvre à l'aide du gaz de balayage (5) contenant du propane et le second perméat (6) est obtenu sous la forme de perméat (6) chargé en propane du gaz de balayage (5),
- où le premier perméat (3) chargé en propane du gaz de balayage (5) et/ou le second perméat (6) chargé en propane du gaz de balayage ou une ou plusieurs parties de ceux-ci sont utilisés dans la formation de la charge réactionnelle (1).

2. Procédé selon la revendication 1, selon lequel la charge réactionnelle (1) présente 45 à 95 pour cent en volume de propane et 1 à 50 pour cent en volume d'hydrogène.

3. Procédé selon la revendication 1 ou 2, selon lequel le mélange de composants (2) et la première charge séparatrice présentent 40 à 60 % en volume de propane, 20 à 30 % en volume de propylène et 25 à 35 % en volume d'hydrogène.

4. Procédé selon l'une quelconque des revendications précédentes, selon lequel le premier rétentat et la seconde charge séparatrice présentent 10 à 20 pour cent en volume d'hydrogène.

5. Procédé selon l'une quelconque des revendications précédentes, selon lequel le second rétentat présente 5 à 15 pour cent en volume d'hydrogène.

6. Procédé selon l'une quelconque des revendications précédentes, selon lequel le mélange de composants (2) ou sa partie soumise à la première séparation membranaire (40) est soumis(e) à un refroidissement (20) et à une compression (30) avant d'être soumis(e) à la séparation membranaire (40).

7. Procédé selon la revendication 6, selon lequel la compression (30) est effectuée à un niveau de pression de 2 à 38 bars.

8. Procédé selon la revendication 6 ou 7, selon lequel la compression (30) est réalisée à l'aide d'un ou de plusieurs turbocompresseurs à plusieurs étages, en particulier à deux étages de refroidissement intermédiaires ou moins.

9. Procédé selon l'une quelconque des revendications précédentes, selon lequel le second rétentat ou une partie de celui-ci, en particulier à un niveau de pression de 1 à 38 bars, est amené à une ou plusieurs autres étapes de procédé (60).

10. Procédé selon la revendication 9, selon lequel la ou les autres étapes de procédé (60) comprennent un procédé de vapocraquage ou une ou plusieurs étapes de séparation associées au procédé de vapocraquage.

11. Procédé selon l'une quelconque des revendications précédentes, selon lequel seule la première séparation membranaire (40) est mise en œuvre à l'aide du gaz de balayage (5) contenant du propane et seul le premier perméat (3) est obtenu sous la forme de perméat (3) chargé en propane du gaz de balayage (5) ou selon lequel seule la seconde séparation membranaire (50) est mise en œuvre à l'aide du gaz de balayage (5) contenant du propane et seul le second perméat (6) est obtenu sous la forme de perméat (6) chargé en propane du gaz de balayage (5), où le perméat (3, 6) respectif non chargé en propane du gaz de balayage (5) ou une partie de celui-ci est brûlé dans un ou plusieurs réacteurs utilisés pour la déshydrogénation du propane (10) pour son/leur chauffage.

12. Procédé selon la revendication 11, selon lequel le perméat (3, 6) respectif non chargé en propane du gaz de balayage (5) est fourni dans la séparation membranaire (40, 50) à un niveau de pression de 3 à 7 bars.

13. Installation de production d'un produit à base de propylène (9), comportant un ou plusieurs réacteurs, lesquels sont conçus pour, à l'aide d'une déshydrogénation de propane (10) à laquelle une charge réactionnelle (1) contenant du propane et de l'hydrogène est soumise, fournir un mélange de composants (2) contenant du propane, du propylène et de l'hydrogène,
- des moyens, lesquels sont conçus pour soumettre le mélange de composants (2) ou une partie de celui-ci, en tant que première charge séparatrice, à une première séparation membranaire (40) au moyen de laquelle un premier perméat (3) enrichi en hydrogène par rapport à la première charge séparatrice, ainsi qu'un premier rétentat (4) appauvri en hydrogène par rapport à la première charge séparatrice et contenant de l'hydrogène, du propane et du propylène sont formés,
- des moyens, lesquels sont conçus pour soumettre le premier rétentat (4) ou une partie de celui-ci, en tant que seconde charge séparatrice, à une seconde séparation membranaire (50), dans laquelle un second perméat (6) contenant au moins la majorité de l'hydrogène de la seconde charge séparatrice et du propane du gaz de balayage, ainsi qu'un deuxième rétentat, contenant au moins la majorité du propane et du propylène de la seconde charge séparatrice sont formés,
- des moyens, lesquels sont conçus pour mettre en œuvre la première séparation membranaire (40) à l'aide d'un gaz de balayage (5) contenant du propane et pour obtenir le premier perméat (3) sous la forme de perméat (3) chargé en propane du gaz de balayage (5) et/ou pour mettre en œuvre la seconde séparation membranaire (50) à l'aide du gaz de balayage (5) contenant du propane et pour obtenir le second perméat (6) sous la forme de perméat (6) chargé en propane du gaz de balayage (5),
- des moyens, lesquels sont conçus pour utiliser le premier perméat (3) chargé en propane du gaz de balayage (5) et/ou le second perméat (3) chargé en propane du gaz de balayage ou une ou plusieurs parties de ceux-ci dans la formation de la charge réactionnelle (1).

14. Installation selon la revendication 13, laquelle est conçue pour mettre en œuvre un procédé selon l'une quelconque des revendications précédentes.
